# EUROPEAN PATENT SPECIFICATION

(11) **EP 0 643 972 B1**
(45) Date of publication and mention of the grant of the patent: **06.03.2002**
(21) Application number: 94202534.7
(22) Date of filing: 16.09.1988
(51) Int. Cl.: A61K 38/10, C07K 14/53

(54) **Use of recombinant colony stimulating factor-1 for the preparation of a medicament for cytomegalovirus infections**
Verwendung von rekombinantem koloniestimulierendem Faktor-1 zur Herstellung eines Medikaments gegen Cytomegalovirusinfektionen
Utilisation d'un facteur-1 stimulant de colonie (CSF-1) recombinant pour la préparation d'un médicament traitant l'infection par le cytomégalovirus

(30) Priority: 22.09.1987 US 99872; 14.09.1988 US 243253
(43) Date of publication of application: 22.03.1995
(62) Divisional of application: 88908821.7
(73) Proprietor: CHIRON CORPORATION, Emeryville, California 94608 (US)
(72) Inventor: Ralph, Peter, Orinda, California (US); Chong, Kong Teck, Union City, California 94587 (US)
(74) Representative: Bizley, Richard Edward

(56) References cited:
- WO-A-86/04587
- WO-A-86/04607
- WO-A-87/03204
- WO-A-87/06954
- US-A- 4 230 697
- US-A- 4 342 828
- NATIONAL LIBRARY OF MEDICINE (NLM), FILE MEDLINE, NLM ACCESSION NO. 84291030, XP002000247 SR PETURSSON ET AL.: "Megakaryocytopoiesis and granulopoiesis after murine cytomegalovirus infection"

## Description

The present invention relates to the use of recombinantly produced human colony stimulating factor-1 (CSF-1) in the treatment of viral infectious disease in mammals, the virus being cytomegalovirus.

The ability of certain factors produced in very low concentration in a variety of tissues to stimulate the growth and development of bone marrow progenitor cells into granulocytes and/or macrophages has been known for nearly 15 years. The presence of such factors in sera, urine samples, and tissue extracts from a number of species is demonstrable using an *in vitro* assay which measures the stimulation of colony formation by bone marrow cells plated in semi-solid culture medium. There is no known *in vivo* assay. Because these factors induce the formation of such colonies, the factors collectively have been called Colony Stimulating Factors (CSF).

More recently, it has been shown that there are at least four subclasses of human CSF proteins which can be defined according to the types of cells found in the resultant colonies. One subclass, CSF-1 results in colonies containing macrophages predominantly. Other subclasses produce colonies which contain both neutrophilic granulocytes and macrophages; which contain predominantly neutrophilic granulocytes; and which contain neutrophilic and eosinophilic granulocytes and macrophages.

There are murine factors analogous to the above human CSFs, including a murine factor called IL-3 which induces colonies from murine bone marrow cells which contain all these cell types plus megakaryocytes, erythrocytes, and mast cells, in various combinations. These CSFs have been reviewed by Dexter, T. M., Nature (1984) 309:746, and Vadas, M. A., et al, J Immunol (1983) 130:793, Clark, S.C., Science (1987) 236:1229, and Sachs, L., Science (1987) 238:1374.

The invention herein is concerned with the use of recombinant proteins which are members of the first of these subclasses, CSF-1. This subclass has been further characterized and delineated by specific radioimmunoassays and radioreceptor assays --e.g., antibodies raised against purified CSF-1 are able to suppress specifically CSF-1 activity, without affecting the biological activities of the other subclasses, and macrophage cell line J774 contains receptors which bind CSF-1 specifically. A description of these assays was published by Das, S. K., et al, Blood (1981) 58:630.

A significant difficulty in putting CSF proteins in general, and CSF-1 in particular, to any useful function has been their unavailability in distinct and characterizable form in sufficient amounts to make their employment in therapeutic use practical or even possible. These deficiencies may be remedied by providing purified human and murine CSF-1 in useful amounts through recombinant techniques.

(A CSF protein of a different subclass, murine and human GM-CSF, has been purified and the cDNAs have been cloned. This protein was shown to be distinct from other CSFs, e.g., CSF-1, by Gough, et al, Nature (1984) 309:763-767. This GM-CSF protein is further described in W087/02060, published 9 April 1987 as being useful to treat cancer patients to regenerate leukocytes after traditional cancer treatment, and to reduce the likelihood of viral, bacterial, fungal and parasitic infection, such as acquired immune deficiency syndrome (AIDS). Murine IL-3 has been cloned by Fung, M. C., et al, Nature (1984) 307:233. See also Yokota, T., et al, Proc Natl Acad Sci (USA) (1984) 81:1070-1074; wong, G.G., et al, Science (1985) 228:810-815; Lee, F., et al, Proc Natl Acad Sci (USA) (1985) 82:4360-4364; and Cantrell, M.A., et al, Proc Natl Acad Sci (USA) (1985) 82:6250-6254.)

Treatment of patients suffering from AIDS with CSF-1, alone or together with erythropoietin and/or an antiviral agent and/or IL-2 is reported in WO87/03204, published 4 June 1987. U.S. Patent No. 4,482,485, issued 13 November 1984 states that CSF can be used for a supporting role in the treatment of cancer. In addition, EP 118,915, published 19 September 1984 reports production of CSF for preventing and treating granulocytopenia and macrophagocytopenia in patients receiving cancer therapy, for preventing infections, and for treating patients with implanted bone marrow. In addition, CSF-1 is reported to stimulate nonspecific tumoricidal activity (Ralph et al, Immunobiol (1986) 172: 194-204). Ralph et al, Cell Immunol (1983) 76: 10-21 reported that CSF has no immediate direct role in activation of macrophages for tumoricidal and microbiocidal activities against firbrosarcoma 1023, lymphoma 18-8, and L. tropica amastigotes. Ralph et al, Cell Immunol (1987) 105: 270-279 reports that the added tumoricidal effect of a combination of CSF-1 and lymphokine on murine sarcoma TU5 targets.

In addition, Warren et al, J Immunol (1986) 137: 2281-2285 discloses that CSF-1 stimulates monocyte production of interferon, TNF and colony stimulating activity. Lee et al, J Immunol (1987) 138: 3019-3022 discloses CSF-1-induced resistance to viral infection in murine macrophages.

WO 86/04587 and WO 86/04607 describe the purification of CSF-1, and its ability to enhance the production of monocytes by stimulating monocyte-precursor cell production. US 4,230,697 and US 4,342,828 describe the use of colony stimulating factor in the treatment of leukopenia and granulocytopenia.

The present invention provides the use of recombinant colony stimulating factor 1 (CSF-1) in the manufacture of a medicament useful for therapeutic treatment of viral infectious disease in mammals, the virus being cytomegalovirus.

Preferably, the medicament is such as to permit administration of the recombinant CSF-1 in conjunction with one or more lymphokines selected from interferon-alpha, interferon-beta, interferon-gamma, IL-2, TNF, IL-1, GM-CSF, or G-CSF.

### Brief Description of the Drawings

Figure 1 shows the DNA and deduced amino acid sequences for a cDNA clone encoding CSF-1.

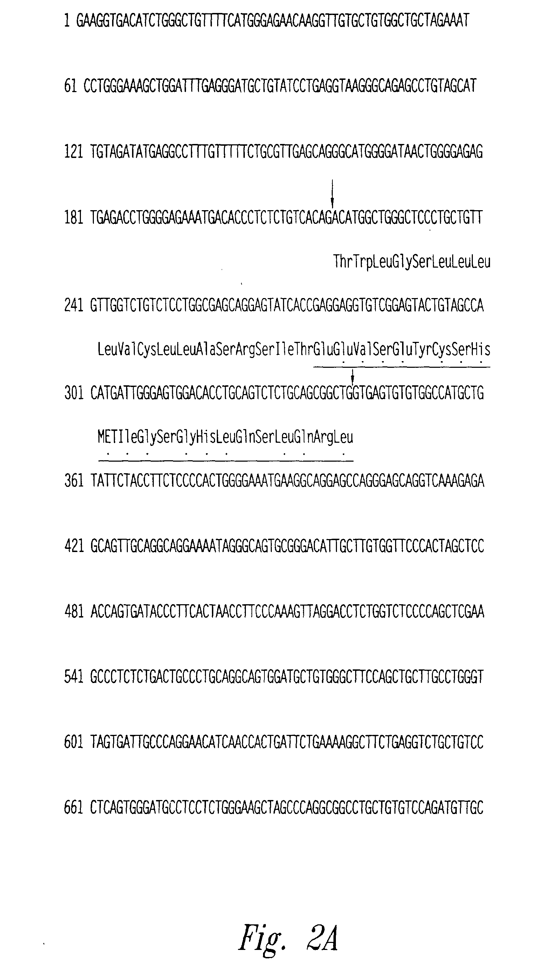

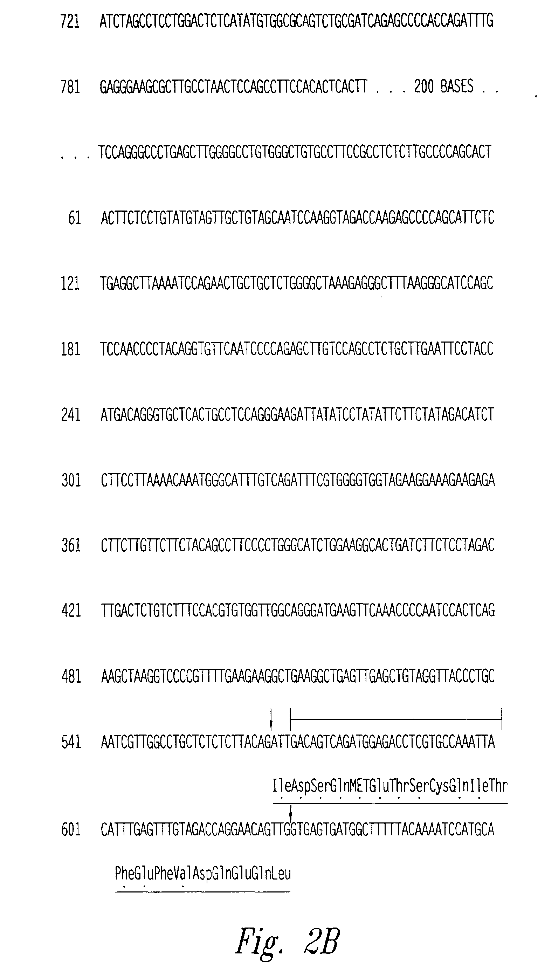

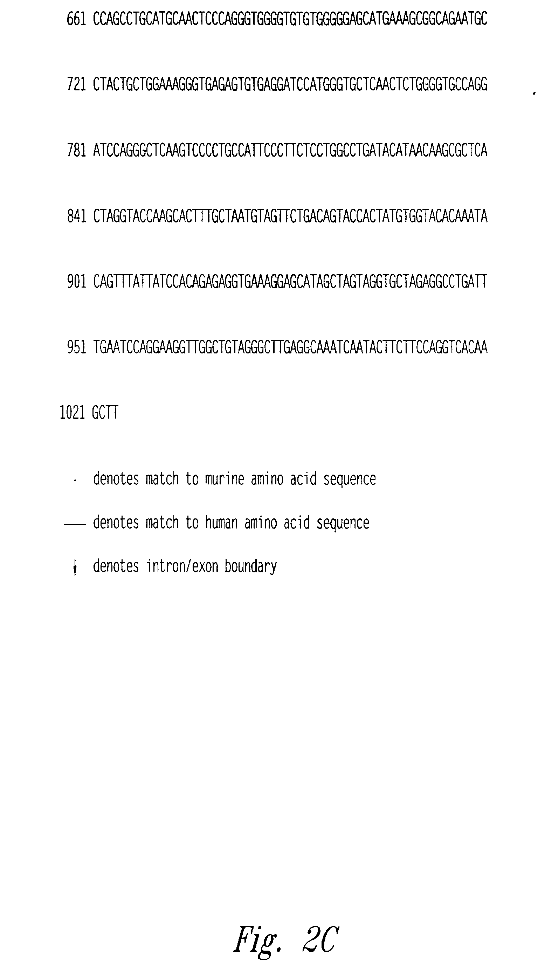

Figure 2 shows the sequenced portion of a 3.9 kb HindIII fragment encoding human CSF-1 sequences and the deduced amino acid sequences for the exon regions.

### A. Definitions

"Colony stimulating factor-1 (CSF-1)" refers to a protein which exhibits the spectrum of activity understood in the art for CSF-1 --i.e., when applied to the standard in vitro colony stimulating assay of Metcalf, D., J Cell Physiol (1970) 76:89, it results in the formation of primarily macrophage colonies. Native CSF-1 is a glycosylated dimer; dimerization may be necessary for activity. Contemplated within the scope of the invention and within the definition of CSF-1 are both the dimeric and monomeric forms. The monomeric form may be converted to the dimer by in vitro provision of intracellular conditions, and the monomer is per se useful as an antigen to produce anti-CSF-1 antibodies.

There appears to be some species specificity: Human CSF-1 is operative both on human and on murine bone marrow cells; murine CSF-1 does not show activity with human cells. Therefore, "human" CSF-1 should be positive in the specific murine radioreceptor assay of Das, S.K., et al, Blood (1981) 58:630, although there is not necessarily a complete correlation. The biological activity of the protein will generally also be inhibited by neutralizing antiserum to human urinary CSF-1 (Das, S.K., et al, supra). However, in certain special circumstances (such as, for example, where a particular antibody preparation recognizes a CSF-1 epitope not essential for biological function, and which epitope is not present in the particular CSF-1 mutein being tested) this criterion may not be met.

Certain other properties of CSF-1 have been recognized more recently, including the ability of this protein to stimulate the secretion of series E prostaglandins, interleukin-1, and interferon from mature macrophages (Moore, R., et al, Science (1984) 223:178). The mechanism for these latter activities is not at present understood, and for purposes of definition herein, the criterion for fulfillment of the definition resides in the ability to stimulate the formation of monocyte/macrophage colonies using bone marrow cells from the appropriate species as starting materials, under most circumstances (see above) the inhibition of this activity by neutralizing antiserum against purified human urinary CSF-1, and, where appropriate for species type, a positive response to the radioreceptor assay. (It is known that the proliferative effect of CSF-1 is restricted to cells of mononuclear phagocytic lineage (Stanley, E.R., The Lymphokines (1981), Stewart, W.E., II, et al, ed, Humana Press, Clifton, NJ), pp. 102-132) and that receptors for CSF-1 are restricted to these cell lines (Byrne, P.V., et al, Cell Biol (1981) 91:848) and placental trophoblasts).

As is the case for all proteins, the precise chemical structure depends on a number of factors. As ionizable amino and carboxyl groups are present in the molecule, a particular protein may be obtained as an acidic or basic salt, or in neutral form. All such preparations which retain their activity when placed in suitable environmental conditions are included in the definition. Further, the primary amino acid sequence may be augmented by derivatization using sugar moieties (glycosylation) or by other supplementary molecules such as lipids, phosphate, acetyl groups and the like, more commonly by conjugation with saccharides. The primary amino acid structure may also aggregate to form complexes, most frequently dimers. Indeed, native human urinary CSF-1 is isolated as a highly glycosylated dimer. Certain aspects of such augmentation are accomplished through post-translational processing systems of the producing host; other such modifications may be introduced in vitro. In any event, such modifications are included in the definition so long as the activity of the protein, as defined above, is not destroyed. It is expected, of course, that such modifications may quantitatively or qualitatively affect the activity, either by enhancing or diminishing the activity of the protein in the various assays.

Further, individual amino acid residues in the chain may be modified by oxidation, reduction, or other derivatization, and the protein may be cleaved to obtain fragments which retain activity. Such alterations which do not destroy activity do not remove the protein sequence from the definition.

Modifications to the primary structure itself by deletion, addition, or alteration of the amino acids incorporated into the sequence during translation can be made without destroying the activity of the protein. Such substitutions or other alterations result in proteins having an amino acid sequence which falls within the definition of proteins "having an amino acid sequence substantially equivalent to that of CSF-1". Indeed, human- and murine-derived CSF-1 proteins have nonidentical but similar primary amino acid sequences which display a high homology.

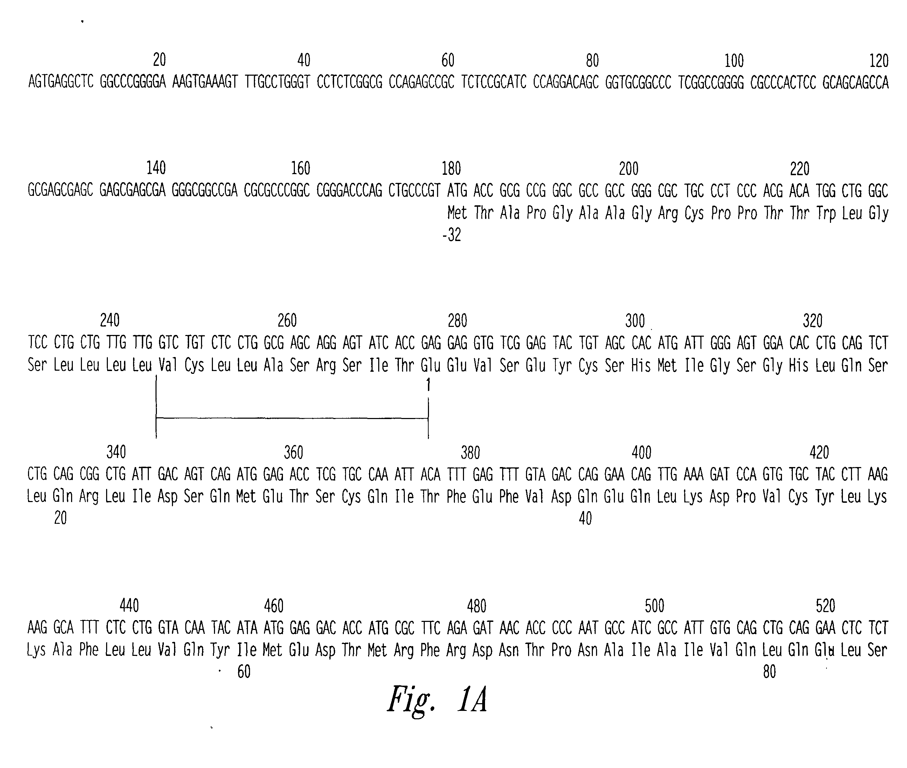

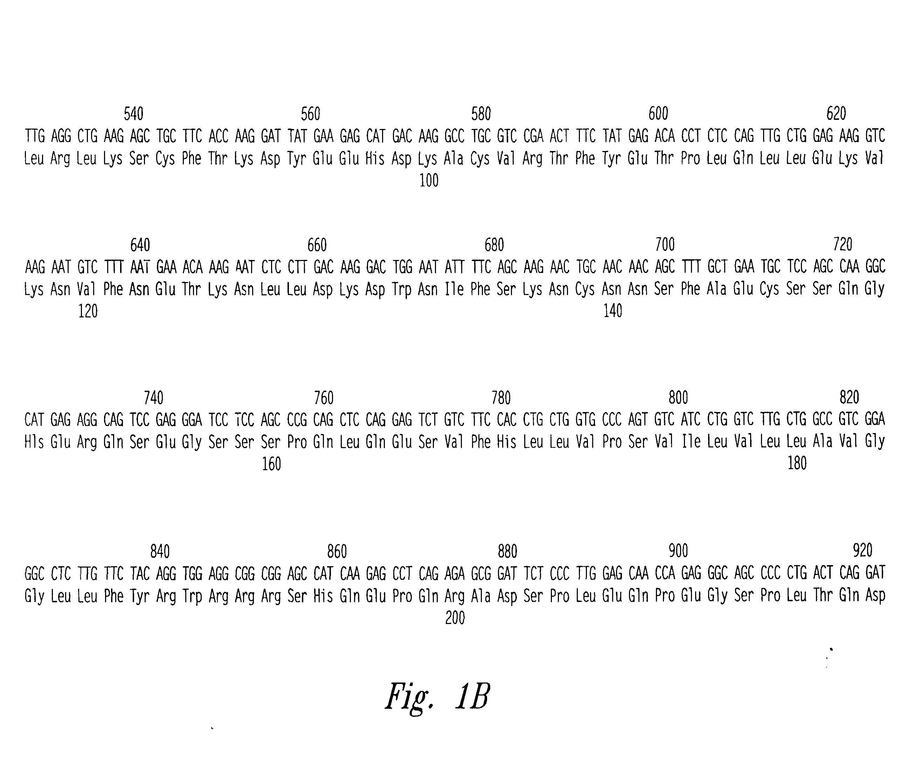

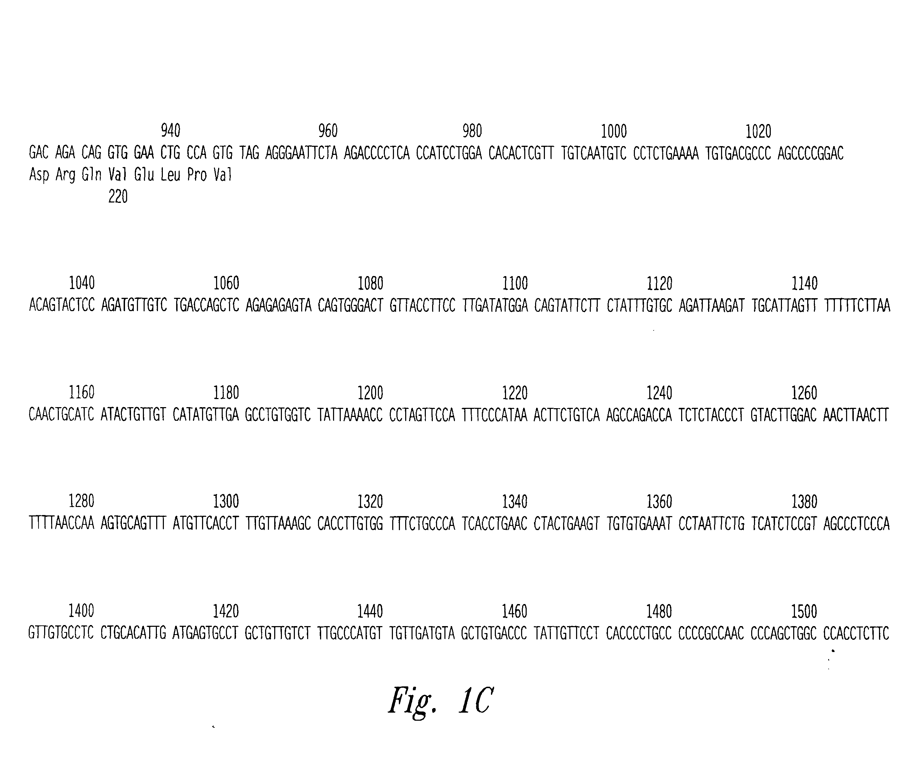

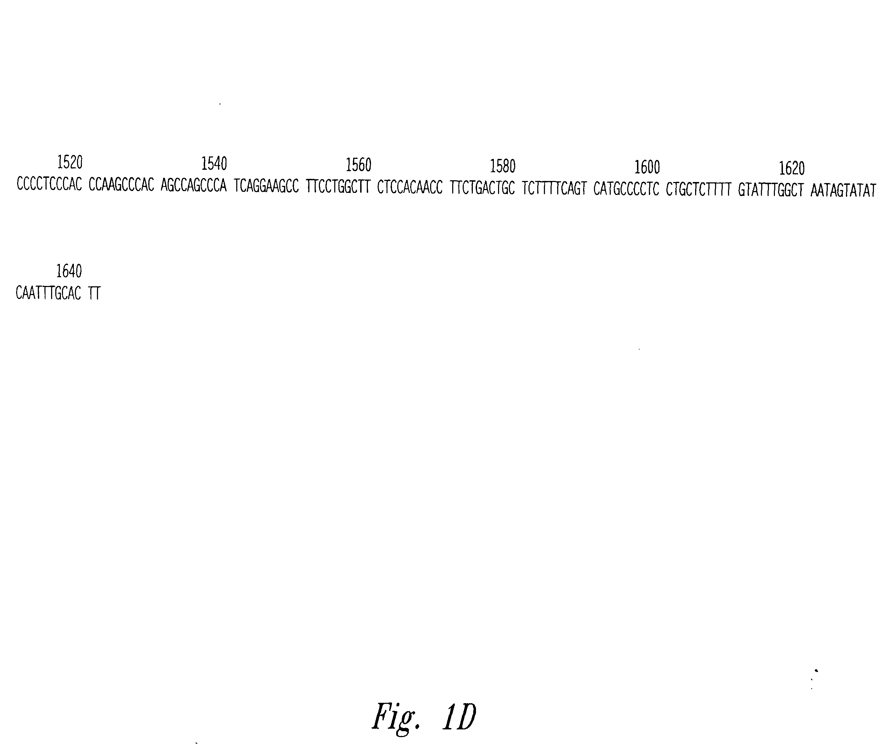

For convenience, the mature protein amino acid sequence of the monomeric portion of a dimeric protein shown in Figure 1, deduced from the cDNA clone illustrated herein, is designated mCSF-1 (mature CSF-1). Figure 1 shows the presence of a 32 residue putative signal sequence, which is presumably cleaved upon secretion from mammalian cells; mCSF-1 is represented by amino acids 1-224 shown in that figure. Specifically included in the definition of human CSF-1 are muteins which monomers and dimers are mCSF-1 and related forms of mCSF-1, designated by their differences from mCSF-1. CSF-1 derived from other species may fit the definition of "human" CSF-1 by virtue of its display of the requisite pattern of activity as set forth above with regard to human substrate.

Also for convenience, the amino acid sequence of mCSF-1 will be used as a reference and other sequences which are substantially equivalent to this in terms of CSF-1 activity will be designated by referring to the sequence shown in Figure 1. The substitution of a particular amino acid will be noted by reference to the amino acid residue which it replaces. Thus, for example, ser₉₀CSF-1 includes the protein which has the sequence shown in Figure 1 except that the amino acid at position 90 is serine rather than cysteine. Deletions are noted by a ∇ followed by the number of amino acids deleted from the N-terminal sequence, or by the number of amino acids remaining when residues are deleted from the C-terminal sequence, when the number is followed by a minus sign. Thus, ∇4CSF-1 includes the CSF-1 of Figure 1 wherein the first 4 amino acids from the N-terminus have been deleted; ∇130- refers to CSF-1 wherein the last 94 amino acids following amino acid 130 have been deleted. Illustrated below are a number of CSF-1 proteins, for example, asp₅₉CSF-1, which contains an aspartic residue encoded by the gene (Figure 1) at position 59 rather than the tyrosine residue encoded by the cDNA, and ∇158-CSF-1 (hereinafter 158), which comprises only amino acids 1-158 of mCSF-1.

"Operably linked" refers to juxtaposition such that the normal function of the components can be performed. Thus, a coding sequence "operably linked" to control sequences refers to a configuration wherein the coding sequence can be expressed under the control of these sequences.

"Control sequences" refers to DNA sequences necessary for the expression of an operably linked coding sequence in a particular host organism. The control sequences which are suitable for procaryotes, for example, include a promoter, optionally an operator sequence, a ribosome binding site, and possibly, other as yet poorly understood, sequences. Eucaryotic cells are known to utilize promoters, polyadenylation signals, and enhancers.

"Effective amount" signifies an amount effective to perform the function specified, such as to kill tumors or reduce tumor burden or prevent or cure infectious diseases.

"Therapeutic treatment" indicates treating after the disease is contracted, whereas "prophylactic" treatment indicates treating before the disease is contracted.

"Mammals" indicates any mammalian species, and includes rabbits, mice, dogs, cats, primates and humans, preferably humans.

"Expression system" refers to DNA sequences containing a desired coding sequence and control sequences in operable linkage, so that hosts transformed with these sequences are capable of producing the encoded proteins. In order to effect transformation, the expression system may be included on a vector; however, the relevant DNA may then also be integrated into the host chromosome.

As used herein "cell", "cell line", and "cell culture" are used interchangeably and all such designations include progeny. Thus "transformants" or "transformed cells" includes the primary subject cell and cultures derived therefrom without regard for the number of transfers. It is also understood that all progeny may not be precisely identical in DNA content, due to deliberate or inadvertent mutations. Mutant progeny which have the same functionality as screened for in the originally transformed cell are included. Where distinct designations are intended, it will be clear from the context.

### B. General Description

The CSF-1 proteins used according to the invention are capable both of stimulating monocyte-precursor/ macrophage cell production from progenitor marrow cells, thus enhancing the effectiveness of the immune system, and of stimulating such functions of these differentiated cells as the secretion of lymphokines in the mature macrophages.

Enhanced amounts of previously differentiated macrophages could be supplied to subjects in order to supplement those of the indigenous system, which macrophages are produced by *in vitro* culture of bone marrow or other suitable preparations followed by treatment with CSF-1. These preparations include those of the patient's own blood monocytes, which can be so cultured and returned for local or systemic therapy.

The ability of CSF-1 to stimulate production of lymphokines by macrophages and to enhance their ability to kill target cells makes CSF-1 directly useful in treatment of infections.

CSF-1 stimulates the production of interferons by murine-derived macrophage (Fleit, H.B., et al, J Cell Physiol (1981) 108: 347), and human, partially purified, CSF-1 from MIAPaCa cells stimulates the poly(I):poly (C) - induced production of interferon and TNF from human monocytes as illustrated in co-owned PCT publication WO 86/04607, published 14 August 1986. In addition, CSF-1 stimulates the production of myeloid CSF by human blood monocytes.

The ability of murine cells to attack infectious organisms, including viruses such as cytomegalovirus (CMV), is enhanced by CSF-1. (Murine CSF-1 is inconsistently reported to stimulate murine macrophage to be cytostatic to P815 tumor cells (Wing, E.J., et al, J Clin Invest (1982) 69: 270) or not to kill other leukaemia targets (Ralph, P, et al, Cell Immunol (1983) 76: 10). Nogawa, R.T., et al, Cell Immunol (1980) 53: 116, report that CSF-1 may stimulate macrophage to ingest and kill yeast).

Thus, CSF-1 can be used to destroy the invading organisms indirectly by stimulation of macrophage secretions and activity.

The CSF-1 used in the invention may be formulated in conventional ways standard in the art for the administration of protein substances. Administration by injection is preferred; formulations include solutions or suspensions, emulsions, or solid composition for reconstitution into injectables. Suitable excipients include, for example, Ringer's solution, Hank's solution, water, saline, glycerol, dextrose solutions, and the like. In addition, the CSF-1 of the invention may be preincubated with preparations of cells in order to stimulate appropriate responses, and either the entire preparation or the supernatant therefrom introduced into the subject. As shown hereinbelow, the materials produced in response to CSF-1 stimulation by various types of blood cells are effective against desired targets, and the properties of these blood cells themselves to attack invading viruses or neoplasms may be enhanced. The subject's own cells may be withdrawn and used in this way, or, for example, monocytes or lymphocytes from another compatible individual employed in the incubation.

The complete coding sequence for human CSF-1 is now available and expression vectors applicable to a variety of host systems have been constructed and the coding sequence expressed. See, for example, PCT publication WO 86/04607, supra; Kawasaki, E.S., et al Science (1985) 230:291-296; Ladner et al, (1987) EMBO J 6(9):2693-2698, WO 88/03173, published 5 May 1988, and EP 0,272,779, published 29 June 1988.

The variety of hosts available, along with expression vectors suitable for such hosts, permits a choice among posttranslational processing systems, and of environmental factors providing conformational regulation of the protein thus produced. Thus, the availability of this information provides the CSF-1 protein in sufficient quantity for application in the various therapies discussed herein.

In the particular case of human CSF-1, evidence now accumulating indicates that considerable deletion at the C-terminus of the protein may occur under both recombinant and native conditions, and that the activity of the protein is still retained. It appears that the native proteins isolated may be in some sort of C-terminal truncated form or mixtures thereof, and may exhibit variable C-terminal processing. The activity of these "truncated" forms is clearly established by their deliberate production. The mutein produced from DNA encoding SCSF/C∇150 (hereinafter 150) for example, is fully active in assays for CSF-1, as is that produced from cDNA encoding LCSF C∇190 (hereinafter 190), LCSF/C∇221 (hereinafter 221), asp₅₉LCSF/N∇3C∇221, (hereinafter N∇3C∇221). These muteins are described in copending European Publication No. 272,779, published 29 June 1988, and PCT publication WO 88/03173, published 5 May 1988.

### C. Preferred Embodiments

The recombinant CSF-1 is considered to be a set of muteins which have similar but not necessarily identical primary amino acid sequences, all of which exhibit, or are specifically cleavable to a mutein which exhibits, the activity pattern characteristic of CSF-1-i.e., they are capable of stimulating bone marrow cells to differentiate into monocytes, preponderantly, and, within the limitations set forth in the Definitions section above, are immunoreactive with antibodies raised against native CSF-1 and with the receptors associated with CSF-1 activity. Certain embodiments of these muteins are, however, preferred.

The primary sequences shown in Figure 1 and in Figure 4 of Ladner et al, supra, for mature CSF-1 have the required activity, and are, of course, among the preferred embodiments. Also preferred are muteins wherein certain portions of the sequence have been altered by either deletion of, or conservative substitution of, one or more amino acids in mature CSF-1. By a "conservative" amino acid substitution is meant one which does not change the activity characteristics of the protein, and in general is characterized by chemical similarity of the side chains of the two residues interchanged. For example, acidic residues are conservatively replaced by other acidic residues, basic by basic, hydrophobic by hydrophobic, bulky by bulky, and so forth. The degree of similarity required depends, of course, on the criticality of the amino acid for which substitution is made, and its nature. Thus, in general, preferred substitutions for cysteine residues are serine and alanine; for aspartic acid residues, glutamic acid; for lysine or arginine residues, histidine; for leucine residues, isoleucine, or valine; for tryptophan residues, phenylalanine or tyrosine; and so forth.

Regions of the CSF-1 protein which are most tolerant of alteration include those regions of known low homology between human and mouse species (residues 15-20 and 75-84); regions which confer susceptibility to proteolytic cleavage (residues 51 and 52 and residues 191-193); cysteine residues not participating in disulfide linkages, or other residues which are not absolutely essential for activity (residues 159-224). It also appears residues 151-224 are not essential.

Therefore, particularly preferred are those CSF-1 muteins characterized by the deletion or conservative substitution of one or more amino acids and/or one or more sequences of amino acids between positions 159 and 224 inclusive of mCSF-1 or positions 151-224 inclusive. In particular, ∇158CSF-1 has CSF activity comparable to that of the native protein, even if a limited number of additional amino acid residues not related to CSF-1 are included at the truncated C-terminus; ∇150CSF-1 has similar activity. Indeed, the native protein is reported to have a molecular weight of 14-15 kd (as opposed to the 26 kd predicted from the cDNA sequence) and the hydrophobicity deduced from the recombinant (predicted) amino acid sequence corresponds to a transmembrane region normally susceptible to cleavage. It may, therefore, be that the truncated version corresponds in a rough way to the CSF-1 as isolated.

Also preferred are CSF-1 proteins which comprise the amino acid sequences containing the first 3-150 or 4-150 amino acids of SCSF or LCSF and the C-terminal deletions, such as, LCSF/C∇221 and asp₅₉LCSF/N∇3C∇221.

Also preferred are muteins characterized by the deletion or conservative substitution of one or more of the amino acids at positions 51 and 52 and/or positions 191, 192 and 193 of mCSF-1. Especially preferred is gln₅₂CSF-1; a corresponding proline substitution is not conservative, and does not yield an active CSF. Since they represent regions of apparently low homology, another preferred set of embodiments is that characterized by the deletion or conservative substitution of one or more of the amino acids at positions 15-20 and/or positions 75-84 of mCSF-1. Also preferred are those muteins characterized by the deletion or conservative substitution of the cysteine residue at any position not essential for disulfide bond formation. Also preferred are those muteins characterized by the deletion or substitution of the tyrosine residue at position 59 of mCSF-1; particularly, substitution by an aspartic acid residue.

### D. Cloning and Expression of Human CSF-1

PCT publication WO 86/04607, supra, illustrates the methods used in obtaining the coding sequence for human CSF-1, for disposing this sequence in expression vectors, and for obtaining expression of the desired protein. The teaching of this publication is specifically incorporated herein by reference.

The full length cDNA is 1.64 kb and encodes a mature CSF-1 protein of 224 amino acids. The clone was designated CSF-17 with Cetus depository number CMCC 2347 and was deposited with the ATCC (ATCC 53149) on 14 June 1985. The plasmid bearing the CSF-1 encoding DNA was designated pcCSF-17.

### D.1 Mutein-Encoding Sequences

Modifications were made of the pcCSF-17 inserts to provide corresponding plasmids encoding muteins of the mCSF-1 protein. For site-specific mutagenesis, pcCSF-17 and M13mp18 were digested with the same restriction enzyme excising the appropriate region of the CSF-1 coding sequence, and the excised sequence was ligated into the M13 vector. Second strand synthesis and recovery of the desired mutated DNA use the following oligonucleotide primers:
for pro₅₂CSF-1, 5'-TACCTTAAACCGGCATTTCTC-3', which creates a new HpaII site at codons 52-53;
for gln₅₂CSF-1, 5'-TACCTTAAACAGGCCTTTCTC-3', which creates a new StuI site at codons 52-53;
for asp₅₉CSF-1 5'-GGTACAAGATATCATGGAG-3', which creates a new EcoRV site at codons 59-60.

After second strand extension using Klenow, the phage were transformed into E. coli DG98 and the resulting plaques screened with kinased labeled probe. After plaque purification, the desired mutated inserts were returned to replace the unmutated inserts in pcCSF-1, yielding pCSF-pro₅₂, pCSF-gln₅₂, and pCSF-asp₅₉, respectively.

Plasmids containing three deletion mutants which encode ∇158CSF-1 were also prepared: pCSF-Bam, pCSF-BamBcl, and pCSF-BamTGA. For pCSF-Bam, pcCSF-17 was digested with BamHI, and the upstream BamHI/BamHI fragment of the coding region was isolated and religated to the vector fragment. The ligation mixture was transformed into E. coli MM294 and plasmids with the correct orientation were isolated. The resulting pCSF-Bam encodes 158 amino acids of the CSF-1 protein fused to six residues derived from the vector at the C-terminus: Arg-His-Asp-Lys-Ile-His.

For pCSF-BamBcl, which contains the entire CSF-1 encoding sequence, except that the serine at position 159 is mutated to a stop codon, the coding sequence was excised from pcCSF-17 and ligated into M13 for site-specific mutagenesis using the primer: 5'-GAGGGATCCTGATCACCGCAGCTCC-3'. This results in a new BclI site at codons 159-160. The mutated DNA was excised with BstXI/EcoRI and ligated into the BstXI/EcoRI digested pcCSF-17, the ligation mixture was transformed into E. coli DG105, a dam host, and the plasmid DNA isolated.

For pCSF-BamTGA, in which the codons downstream of the 159-stop are deleted, pCSF-BamBcl was digested with XhoI and BclI, and the insert ligated into XhoI/BamHI digested pcCSF-17.

In addition, pCSF-Gly₁₅₀, which contains a TGA stop codon instead of histidine at position 151, was prepared from the pcCSF-17 insert by site-specific mutagenesis using the appropriate primer, as described above.

### D.2 Expression and Biological Assay of CSF-1

The expression of plasmid DNA from CSF-17 (pcCSF-17) in COS-7 cells was confirmed and quantitated using the bone marrow proliferation assay, the colony stimulation assay and the radioreceptor assay as taught in WO 86/04607, supra. It will be recalled that the specificity of the bone marrow proliferation assay for CSF-1 resides only in the ability of CSF-1 antiserum to diminish activity; that for the colony stimulation assay, in the nature of the colonies obtained. Both assays shoved CSF-1 production to be of the order of several thousand units per ml.

### D.3 Expression of Muteins

In a similar manner to that taught for pcCSF-17, the mutein-encoding plasmids were transfected into COS-A2 cells and transient expression of CSF-1 activity was assayed by the bone marrow proliferation assay and by radioimmunoassay using anti-CSF antibodies. The expression product of pCSF-pro52 was inactive, indicating that, as expected, substitution by proline is not conservative. All other muteins showed activity in both assays as shown by the results below:

| Expression of CSF-1 Constructs in COS Cells | | | |
|---|---|---|---|
| CSF-1 Plasmid | Radioimmunoassay (units/ml) | Bone Marrow Assay (units/ml) | |
| | | Proliferation (units/ml) | Colony |
| pcCSF-17 | 3130 | 2798 | 11,100 |
| | 3080 | 3487 | 9750 |
| | 3540 | 3334 | 11,500 |
| | | | |
| pCSF-pro52 | 54.8 | <25 | <100 |
| | 51.9 | <25 | <100 |
| | 45.3 | <25 | <100 |
| | | | |
| pCSF-gln52 | 1890 | 2969 | 6200 |
| | 2250 | 2308 | 5500 |
| | 1910 | 2229 | 4400 |
| | | | |
| pCSF-asp59 | 3210 | 3381 | 9000 |
| | 4680 | 3417 | 6800 |
| | 3470 | 2812 | 10,600 |
| | | | |
| pCSF-Bam | 9600 | 8048 | 22,600 |
| | 8750 | 8441 | 21,900 |
| | 8400 | 10,995 | 21,700 |
| | | | |
| pCSF-BamBcl | 8800 | -- | 26,000 |
| | 10,700 | -- | 21,600 |
| | 15,450 | -- | 24,200 |
| | | | |
| pCSF-BamTGA | 8450 | -- | 22,600 |
| | 7550 | -- | 23,200 |
| | 9700 | -- | 20,000 |
| | | | |
| pCSF-Gly150 | 26,850 | -- | 55,710 |

### D.4 Murine CSF-1

An intronless DNA sequence encoding murine CSF-1 is taught in WO 86/04607, supra.

This murine CSF-1 is prepared using a murine fibroblast cell line which produces large amounts of CSF-1. The L-929 line, obtainable from ATCC, is used as a source for mRNA in order to produce a cDNA library. Using oligomeric probes constructed on the basis of the known murine N-terminal and CNBr-cleaved internal peptide sequence, this cDNA library is probed to retrieve the entire coding sequence for the murine form of the protein. Murine CSF-1 is believed to be approximately 80% homologous to the human material because of the homology of the N-terminal sequences, the ability of both human and murine CSF-1 preparations to stimulate macrophage colonies form bone marrow cells, and limited cross-reactivity with respect to radioreceptor and radioimmunoassays (Das, S.K., et al, Blood (1981) 58: 630). The preparation of the cDNA, cloning and expression of murine CSF-1 are as described in WO 86/04607, supra.

### E. Activity of CSF-1

The activity of CSF-1 was determined in the following Examples using partially purified MIAPaCa CSF-1, murine L cell CSF-1, CV-1-produced recombinant material or *E. coli*-produced human CSF-1.

CSF-1 was demonstrated to inhibit the growth of cytomegalovirus in mice.

The following Examples are illustrative, not limiting

### Examples

### In Vitro Stimulation of Murine Antiviral Activity

Adherent murine thioglycolate-elicited macrophages were incubated with CSF-1 for 3 days and infected with VSV overnight (Lee, M.T., et al (1987) J Immunol 138:3019-3022). The following table shows crystal violet staining as measured by absorbance at 550 nm of cells remaining adherent.

| Treatment | Absorbance (mean)(S.D.) |
|---|---|
| Medium/No virus | 0.346±0.02 |
| Medium + virus | 0.170±0.02 |
| CSF-1, 1000 U/ml + virus | 0.264±0.02 |
| CSF-1, 2000 U/ml + virus | 0.356±0.04 |

CSF-1 treated cells, therefore, showed protection of the macrophage against VSV.

### In Vivo Treatment of CMV Infection with CSF-1

Outbred CD-1 mice were treated with the CSF-1 (158) produced from the CV-1 cell line at doses of 400 µg/kg, intraperitoneally, once a day for five days, starting two days before infection with a sub-lethal dose of cytomegalovirus (CMV). Mice were sacrificed on the third day after infection and the extent of viral replication in target organs such as the spleen was evaluated by plaque assay. The results showed that mice treated with CSF-1 have significantly lowered (57.8% reduction in) spleen viral titer compared to the saline-treated control mice, indicating that CMV infection is less severe in CSF-1-treated mice.

Separately, CSF-1 produced in E. coli (N∇3C∇221) has been tested in a lethal murine CMV infection model in outbred CD-1 mice (this is in contrast to the above experiment using sub-lethal doses of CMV, in which organ titers were monitored). When CSF-1 was administered intraperitoneally to mice at 3-4 mg/kg (single dose given per mouse) 24 hours before viral challenge, there was a significant increase in survival as compared to saline-treated control.

Thus, CSF-1 may be used alone or in combination with another lymphokine in the treatment of viral infections in general, and in particular, may be beneficial in immunosuppressive viral infection such as acquired immune deficiency syndrome (AIDS).

The preferred dosage range is about 0.4-4 mg/kg CSF-1 per mouse per day.

### F. Formulation and Dosage of CSF-1

The recombinantly produced human CSF-1 may be formulated for administration using standard pharmaceutical procedures. Ordinarily CSF-1 will be prepared in injectable form, and may be used either as the sole active ingredient, or in combination with other proteins or other compounds having complementary or similar activity. Such other compounds may include alternate antitumor, e.g., chemotherapeutic, agents such as adriamcyin, or lymphokines, such as IL-1, -2, -3, and -4, alpha-, beta-, and gamma-interferons, CSF-GM and CSF-G, and tumor necrosis factor. The effect of the CSF-1 active ingredient may be augmented or improved by the presence of such additional components. As described above, the CSF-1 may interact in beneficial ways with appropriate blood cells, and the compositions of the invention therefore include incubation mixtures of such cells with CSF-1, optionally in the presence of additional lymphokines or cytokines. Either the supernatant fractions of such incubation mixtures, or the entire mixture containing the cells as well, may be used. Staggered timing may be preferred for some combinations, such as CSF-1 followed one to two days later by γ-interferon.

The CSF-1 described herein is generally administered therapeutically for CMV infection treatment in amounts of between 0.01-1 mg/kg per day, whether single bolus administration or fractionated over 24 hr.

The following deposits have been made:

| Plasmid | CMCC No. | ATCC No. | Deposit Date |
|---|---|---|---|
| pHCSF-1 | | 40177 | 2 April 1985 |
| pHCSF-1a | 2312 | 40185 | 21 May 1985 |
| CSF-17 | 2347 | 53149 | 14 June 1985 |
| pLCSF221A | 3095 | 67390 | 14 April 1987 |
| pCSF-asp59 | 2705 | 67139 | 19 June 1986 |
| pCSF-gln52 | 2708 | 67140 | 19 June 1986 |
| pCSF-pro52 | 2709 | 67141 | 19 June 1986 |
| pCSF-Bam | 2710 | 67142 | 19 June 1986 |
| pCSF-BamBcl | 2712 | 67144 | 19 June 1986 |
| pCSF-Gly150 | 2762 | 67145 | 19 June 1986 |

These deposits were made under the provisions of the Budapest Treaty on the International Recognition of the Deposit of Microorganisms for the Purposes of Patent Procedure and the Regulations thereunder (Budapest Treaty). This assures maintenance of a viable culture for 30 years from date of deposit. The deposits will be made available by ATCC under the terms of the Budapest Treaty, and subject to an agreement between Applicants and ATCC which assures permanent and unrestricted availability upon issuance of the pertinent US patent. The Assignee herein agrees that if the culture on deposit should die or be lost or destroyed when cultivated under suitable conditions, it will be promptly replaced upon notification with a viable specimen of the same culture. Availability of the deposits is not to be construed as a license to practice the invention in contravention of the rights granted under the authority of any government in accordance with its patent laws.

These deposits were made for the convenience of the relevant public and do not constitute an admission that a written description would not be sufficient to permit practice of the invention or an intention to limit the invention to these specific constructs. Set forth hereinabove is a complete written description enabling a practitioner of ordinary skill to duplicate the constructs deposited and to construct alternative forms of DNA, or organisms containing it, which permit practice of the invention as claimed.

## Claims

1. Use of recombinant colony stimulating factor 1 (CSF-1) in the manufacture of a medicament useful for therapeutic treatment of viral infectious disease in mammals, the virus being cytomegalovirus.

2. The use of claim 1, wherein the medicament is such as to permit administration of the recombinant CSF-1 in conjunction with one or more lymphokines selected from interferon-alpha, interferon-beta, interferon-gamma, IL-2, TNF, IL-1, GM-CSF, or G-CSF.

## Patentansprüche

1. Verwendung von rekombinantem koloniestimulierendem Faktor 1 (CSF-1) zur Herstellung eines Arzneimittels für die Verwendung in der therapeutischen Behandlung von viralen Infektionskrankheiten in Säugern, wobei das Virus das Cytomegalievirus ist.

2. Verwendung nach Anspruch 1, wobei das Arzneimittel ein Arzneimittel ist, das die Verabreichung des rekombinanten CSF-1 in Verbindung mit einem oder mehreren Lymphokinen ausgewählt aus Interferon-α, Interferon-β, Interferon-γ, IL-2, TNF, IL-1, GM-CSF oder G-CSF erlaubt.

## Revendications

1. Utilisation d'un facteur de stimulation des colonies 1 recombinant ("CSF-1") pour la fabrication d'un médicament destiné au traitement thérapeutique d'une maladie infectieuse virale chez un mammifère, le virus étant un cytomégalovirus.

2. Utilisation selon la revendication 1, **caractérisée en ce que** le médicament est tel qu'il permet l'administration du CSF-1 recombinant conjointement avec au moins une lymphokine sélectionnée parmi interféron-alpha, interféron-bêta; interférongamma, IL-2, TNF, IL-1, GM-CSF ou G-CSF.
